# EUROPEAN PATENT APPLICATION

(11) **EP 3 760 268 A1**
(43) Date of publication of application: **06.01.2021**
(21) Application number: 20191578.2
(22) Date of filing: 19.04.2011
(51) Int. Cl.: A61M 25/00

(54) **LOW PROFILE GUIDING CATHETER FOR NEUOROVASCULAR APPLICATIONS**

(30) Priority: 19.04.2010 US 32578410 P
(62) Divisional of application: 11716775.9
(71) Applicant: Micrus Endovascular LLC, San Jose, California 95131 (US)
(72) Inventor: WILLIAMS,, Eric, Miramar, FL 33026 (US); HORTON,, Joseph, Lafayette, LA 70503 (US); ECHARRI,, Roberto, Miami, FL 33145 (US)
(74) Representative: Small, Gary James

(57) **Abstract**

A low profile guiding catheter has a non-round shape for use in delivery of multiple microcatheters for treatment of neurovascular defects, such as for treatment of aneurysms. The low profile guiding catheter includes torque transmittal guidance walls that are flexible linearly but not circumferentially, and that are neither collapsible nor kinkable.

## Description

### CROSS-REFERENCE TO RELATED APPLICATION

This application claims the benefit of the priority date of provisional patent application serial no. 61/325,784 filed April 19, 2010 which is expressly incorporated herein by reference in its entirety.

### BACKGROUND OF THE INVENTION

The present invention relates generally to guiding catheters for devices for interventional therapeutic treatment or vascular surgery for treatment of defects in the vasculature, and more particularly relates to a guiding catheter having torque transmittal guidance walls that are flexible linearly but not circumferentially, and that are neither collapsible nor kinkable, for delivering intravascular interventional devices for treatment of defects in the neurovasculature, such as for treatment of aneurysms.

Vascular interventional devices such as vasoocclusive devices are typically placed within the vasculature of the human body by use of a catheter. Vasoocclusive devices are typically either placed within a blood vessel to block the flow of blood through the vessel by forming an embolus, or are placed within an aneurysm stemming from the vessel to form an embolus within the aneurysm. Vasoocclusive devices used for these procedures can also have a wide variety of configurations, and aneurysms have been treated with external surgically placed clips, detachable vasoocclusive balloons and embolus generating vasoocclusive devices such as one or more vasoocclusive coils. The delivery of such vasoocclusive devices have typically been accomplished by a variety of means, including via a catheter in which the device is pushed through an opening at the distal end of the catheter by a pusher to deploy the device. The vasoocclusive devices can be produced in such a way that they will pass through the lumen of a catheter in a linear shape and take on a complex shape as originally formed after being deployed into the area to be treated, such as an aneurysm.

The insertion of a guiding catheter or delivery catheter system into an arterial site remote from the area to be treated is the first step for endovascular treatment, and is one of the most important steps for treatment of defects in the neurovasculature, such as for treatment of aneurysms. Since the puncture site is generally quite remote from the site to be treated, the size of the puncture is often critical. Typically, guiding catheters have had a circular cross-sectional exterior shape. It would be desirable to provide a guiding catheter or delivery catheter having a cross-sectional shape that will reduce the French size equivalent cross-section and to thus reduce the size of the puncture site, while maintaining the advantages of a larger diameter catheter for delivery of a plurality of microcatheters to a treatment site. It would also be desirable to provide a guiding catheter or delivery catheter having a cross-sectional shape that can flex more easily. The present invention meets these and other needs.

### SUMMARY OF THE INVENTION

The present invention provides for a low profile guiding catheter having a non-circular exterior cross sectional shape for use in delivery of multiple microcatheters for treatment of neurovascular defects, such as for treatment of aneurysms. The low profile guiding catheter of the present invention includes torque transmittal guidance walls that are flexible linearly but not circumferentially, and that are neither collapsible nor kinkable.

By changing the shape of the cross-section of a guiding catheter or delivery catheter having a lumen accommodating multiple microcatheters, the guiding catheter or delivery catheter can have a smaller cross-sectional area, and consequently a smaller puncture size. By changing the cross section shape of the lumen of the catheter from a circle to an oval, two 0.017" devices can be accommodated in a 5Fr equivalent guiding catheter that fits a 6Fr introducer system, but has a smaller profile, thus minimizing vessel trauma. The outside catheter shaft structure may be composed of a braid and/or coil construction, with a lubricious inner lumen of PTFE Teflon® to optimize the wire exchange process in the most distal sections of the arteries. The proximal area of the guiding catheter can have an ergonomically designed hub to allow a physician to easily manipulate the catheter, and to insert other medical devices. In one presently preferred embodiment, the guiding catheter includes a segmented, progressively compliant tip design that incorporates a compliant polymeric material to minimize vessel trauma. The exterior of the catheter may be covered with a polymer material to encapsulate a stainless steel and/or platinum braid and/or coil construction. In a currently preferred embodiment, such polymer material includes a lubricious hydrophilic outer coating.

In one of several currently preferred embodiments of the invention, a low profile guiding catheter for neurovascular applications is provided having an elongated wall structure extending along a length of the catheter defining at least one lumen having a low profile, non-circular cross-sectional shape which may flex relatively easily along at least a portion of the length of the catheter. In one presently preferred embodiment of the invention, the low profile catheter may also have guidance walls along at least a portion of the elongated wall structure forming the exterior of the catheter that are linearly flexible, circumferentially relatively inflexible, and resistant to collapse and kinks.

The portion of length of the catheter having a low profile, non-round cross-sectional shape that flexes easily may flex more easily in at least one dimension than an analogous portion of length of a conventional catheter having a circular cross-sectional shape. In a presently preferred embodiment, the non-round cross-sectional shape may be an oval, a parallelogram, or a triangle with rounded corners. In another aspect of the invention, the elongated wall structure may be formed of metallic or plastic braids or coils, or combinations thereof. In one aspect of the invention, the elongated wall structure defines only one lumen. A portion of the elongated wall structure defining an innermost lumen may have a lubricious inner surface to optimize a wire exchange process in distal sections of arteries. The lubricious inner surface may be provided by a coating on the elongated wall structure. The lubricious inner surface may be formed of or include polytetrafluoroethylene (PTFE). The distal end of the catheter may have a segmented progressively compliant distal tip design having a compliant polymeric material therein to minimize vessel trauma. The catheter may have an exterior surface having a polymeric material with a lubricious hydrophilic outer coating to encapsulate the braids. The catheter may have an exterior surface having a polymeric material with a lubricious hydrophilic outer coating to encapsulate the coils. The elongated wall structure may define an innermost lumen configured to fit two or more medical devices therein. At least two microcatheters may be provided and disposed within an innermost lumen of the low profile guiding catheter, for example within a low profile guiding catheter having an oval cross-sectional shape. At least three microcatheters may be provided disposed within an innermost lumen of the low profile guiding catheter, for example within a low profile guiding catheter having a triangular cross-sectional shape with rounded corners. At least four microcatheters may be provided disposed within an innermost lumen of the low profile guiding catheter, for example within a low profile guiding catheter having a parallelogram cross-sectional shape.

In another aspect of the several preferred embodiments of the present invention, a low profile guiding catheter for neurovascular applications is provided having an elongated wall structure extending along a length of the catheter, said wall structure defining at least one lumen having a low profile, non-round cross-sectional shape that flexes easily along at least a portion of the length of the catheter. A guiding catheter according to the invention preferably has a distal end of a first French size and a proximal end configured to fit with a proximal end of a medical device of a second French size that is larger than the first French size. The elongated wall structure may define an innermost lumen configured to fit two or more medical devices therein. At least two microcatheters may also be provided disposed within an innermost lumen of the low profile guiding catheter, for example a guiding catheter having an oval cross-sectional shape.

In yet another aspect of the several aspects of the present invention a method of minimizing trauma during delivery of at least one intravascular interventional device for treatment of defects in a neurovasculature is provided. The method involves inserting a low profile guiding catheter, according to any of the aspects described above, into the neurovasculature and delivering an intravascular interventional device through a lumen defined by the elongated wall structure of the low profile guiding catheter.

These and other aspects and advantages of the invention will become apparent from the following detailed description and the accompanying drawings, which illustrate by way of example the features of the invention.

### BRIEF DESCRIPTION OF THE DRAWINGS

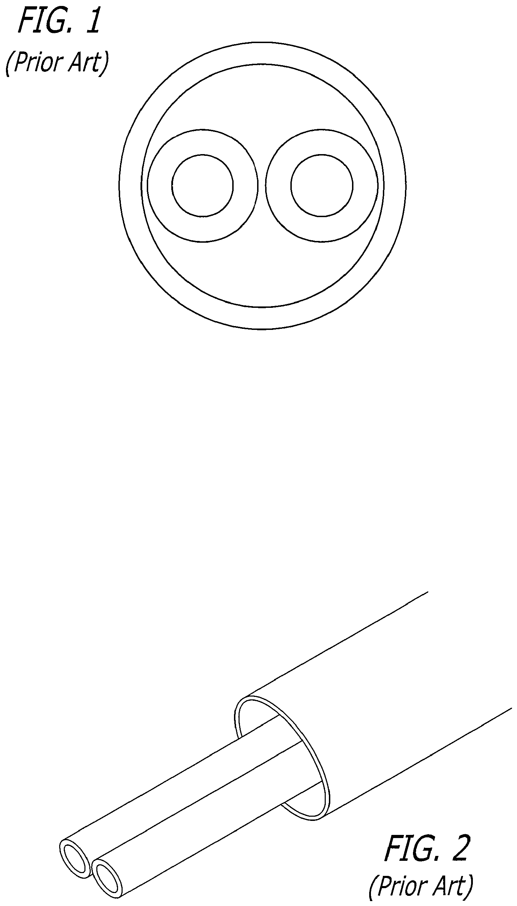

Figure 1 is a front view of a prior art round guiding catheter with two microcatheters.
Fig. 2 is an isometric view of the round guiding catheter of Fig. 1.

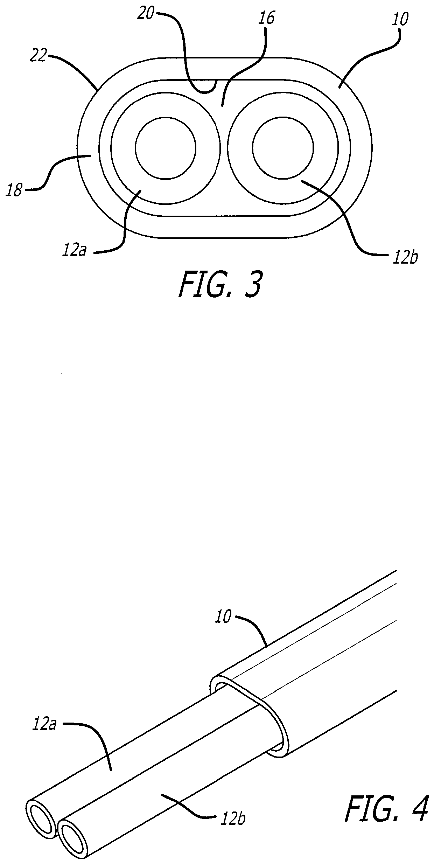

Fig. 3 is a front view of a low profile guiding catheter with two microcatheters, according to an embodiment of the present invention in which the non-round cross-sectional shape is flattened or substantially oval.
Fig. 4 is an isometric view of the low profile guiding catheter with two microcatheters, according to an embodiment of the present invention in which the non-round cross-sectional shape is flattened or substantially oval.
Fig. 5 is a schematic diagram of a cross-section of a flattened guiding catheter with two microcatheters, according to one embodiment of the present invention.
Fig. 6 is a perspective view of a flattened guiding catheter with two microcatheters, according to another embodiment of the present invention in which the distal tip of the guiding catheter has a segmented progressively compliant tip design.

### DETAILED DESCRIPTION OF THE PREFERRED EMBODIMENTS

Referring to the drawings, which are provided by way of example, and not by way of limitation, the present invention provides for a low profile guiding catheter 10 having a non-round shape for use in delivery of multiple microcatheters 12a, 12b for treatment of neurovascular defects, such as for treatment of aneurysms. The low profile guiding catheter of the present invention includes torque transmittal guidance walls 14a, 14b that are flexible linearly but not circumferentially, and that are neither collapsible nor kinkable. By changing the shape of the cross section of the catheter lumen 16 we can accommodate more standard devices into a smaller catheter.

For example, in order to deliver two microcatheters having a 0.017" inner diameter lumen into a brain vasculature, physicians typically select a guiding catheter having a 6Fr or equivalent 0.070" inner diameter lumen. With our invention, by changing the exterior cross section shape of the lumen of the catheter from a circle to an oval, we can accommodate two 0.017" devices into a 5Fr equivalent guide catheter that still fits a 6Fr introducer system but has a smaller profile, thus minimizing vessel trauma. In a currently preferred embodiment, the exterior cross section of the catheter is an oval shape. The specific bending moment or planes of minimum bending force for the low profile guiding catheter can be dependent upon the cross-sectional shape. For example, a low profile guiding catheter having an oval cross-sectional shape will have a bending moment dependent on the ratio of the major and minor axes for a given outer wall thickness. In this manner, the specific bending moments of the low profile guiding catheter may be controlled and modified as desired by changing the dimensions of the catheter cross-sections.

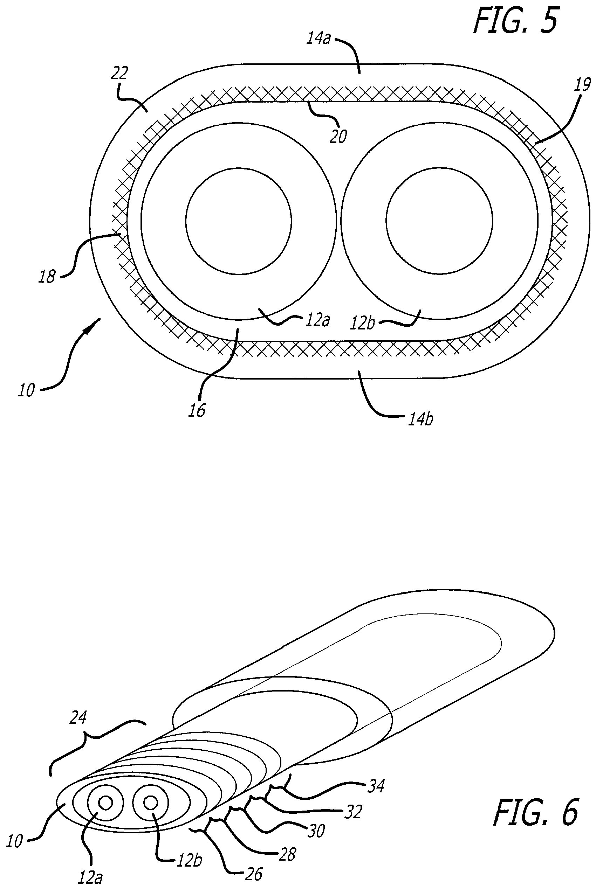

In one preferred embodiment, the catheter shaft preferably is comprised of a combination of a stainless and/or platinum braid 18 and/or coil 19 construction, with a lubricious inner lumen coating 20 of PTFE Teflon® to optimize the wire exchange process in the most distal sections of the arteries. The proximal area (not shown) will have an ergonomically designed hub to allow the physician to easily manipulate the catheter as well as the insertion of other medical devices. As shown in Fig. 6, a segmented progressively compliant tip design 24 incorporates a compliant polymeric material to minimize vessel trauma. For example, according to one embodiment, the segmented progressively compliant tip design 24 may comprises various segments 26, 28, 30, 32, 34 of different materials and/or the segments may be stepped such that the stiffness of the segmented progressively compliant distal tip 24 varies from one segment to the next. The exterior of the catheter 22 is preferably covered with a polymer material to encapsulate the stainless and/or platinum braid 18 and/or coil 19 construction. In a preferred embodiment, such a polymer material will have a lubricious hydrophilic outer coating.

It will be apparent from the foregoing that while particular forms of the invention have been illustrated and described, various modifications can be made without departing from the spirit and scope of the invention. Thus, the invention is not intended to be limited except by the appended claims,

The following is a non-exhaustive list of embodiments which may be claimed:
1. A low profile guiding catheter for neurovascular applications comprising:
   elongated wall structure extending along a length of the catheter defining at least one lumen having a low profile, non-circular cross-sectional shape that flexes easily along at least a portion of the length of the catheter; and
   guidance walls along at least a portion of the elongated wall structure which are linearly flexible, circumferentially relatively inflexible, and resistant to collapse and kinks.
2. The low profile guiding catheter of embodiment 1, wherein the non-circular cross-sectional shape is an oval.
3. The low profile guiding catheter of embodiment 1, wherein the elongated wall structure is further comprised of braided material in said wall.
4. The low profile guiding catheter of embodiment 1, wherein the elongated wall structure is further comprised of coils in said wall.
5. The low profile guiding catheter of embodiment 4, wherein said coils comprise elongate coils, the axis of which is parallel to the longitudinal axis of said wall structure.
6. The low profile guiding catheter of embodiment 1, wherein a portion of the elongated wall structure defining an innermost lumen has a lubricious inner surface to optimize a wire exchange process in distal sections of arteries.
7. The low profile guiding catheter of embodiment 6, wherein the elongated wall structure defines one lumen.
8. The low profile guiding catheter of embodiment 6, wherein the lubricious inner surface is provided by a coating on the elongated wall structure.
9. The low profile guiding catheter of embodiment 8, wherein the lubricious inner surface is provided by polytetrafluoroethylene.
10. The low profile guiding catheter of embodiment 1, wherein said catheter has a distal end with a segmented progressively compliant distal tip design comprising a compliant polymeric material therein chosen to minimize vessel trauma.
11. The low profile guiding catheter of embodiment 3, wherein said catheter has an exterior surface comprising a polymeric material with a lubricious hydrophilic outer coating to encapsulate said braided material.
12. The low profile guiding catheter of embodiment 4, wherein said catheter has an exterior surface comprising a polymeric material with a lubricious hydrophilic outer coating to encapsulate said coils.
13. The low profile guiding catheter of embodiment 1, wherein the elongated wall structure defines an innermost lumen configured to accommodate a plurality of medical devices therein.
14. A low profile guiding catheter for neurovascular applications comprising:
   an elongated outer wall structure extending along a length of the catheter defining at least one lumen having a low profile, non-round cross-sectional shape that flexes relatively easily along at least a portion of the length of the catheter, said guiding catheter having a distal end of a first French size and a proximal end configured to fit with a proximal end of a medical device, the proximal end of the medical device of a second French size that is larger than the first French size.
15. The low profile guiding catheter of embodiment 14, wherein the inner wall of said elongated wall structure defines an inner lumen configured to fit two or more medical delivery microcatheters therein.
16. The low profile guiding catheter of embodiment 15, further comprising at least two microcatheters disposed within an innermost lumen of said low profile guiding catheter.
17. A method of minimizing trauma during delivery of at least one intravascular interventional device for treatment of defects in a neurovasculature, comprising:
   inserting a low profile guiding catheter into the neurovasculature, said low profile guiding catheter including an elongated wall structure extending along a length of the catheter defining at least one lumen having a low profile, non-circular cross-sectional shape that flexes easily along at least a portion of the length of the catheter, and guidance walls along at least a portion of the elongated wall structure, said guidance walls being linearly flexible, circumferentially relatively inflexible, and resistant to collapse and kinks; and
   delivering an intravascular interventional device through a lumen defined by the elongated wall structure of the low profile guiding catheter.

## Claims

1. A low profile guiding catheter for neurovascular applications comprising:
an elongated outer wall structure extending along a length of the catheter defining at least one lumen having a low profile, non-round cross-sectional shape that flexes relatively easily along at least a portion of the length of the catheter, said guiding catheter having a distal end of a first French size and a proximal end configured to fit with a proximal end of a medical device, the proximal end of the medical device of a second French size that is larger than the first French size.

2. The low profile guiding catheter of claim 1, wherein the inner wall of said elongated wall structure defines an inner lumen configured to fit two or more medical delivery microcatheters therein.

3. The low profile guiding catheter of claim 2, further comprising at least two microcatheters disposed within an innermost lumen of said low profile guiding catheter.
